Europäisches Patentamt

European Patent Office

Office européen des brevets

(11) Publication number: 0 348 355
A1

## EUROPEAN PATENT APPLICATION

(21) Application number: 89810473.2

(22) Date of filing: 19.06.89

(51) Int. Cl.⁴: A 41 C 1/10
A 61 N 5/06

(30) Priority: 24.06.88 JP 84244/88

(43) Date of publication of application:
27.12.89 Bulletin 89/52

(84) Designated Contracting States:
BE CH DE FR GB IT LI

(71) Applicant: Gyotoku, Fusako
5-20, 1 chome, Ina
Minou-city Osaka-fu (JP)

(72) Inventor: Gyotoku, Fusako
5-20, 1 chome, Ina
Minou-city Osaka-fu (JP)

(74) Representative: Baggiolini, Raimondo et al
Patent Attorneys Fiammenghi-Fiammenghi-Racheli Via
San Gottardo 15
CH-6900 Lugano (CH)

(54) Maternity binder.

(57) In view of the fact that recently in the medical and other fields it has been clarified that far-infrared rays exhibit good performance characteristics when applied to human body, the invention is intended to arrange that far-infrared rays (5) are radiated toward the abdomen of the pregnant woman through the provision of an improved maternity binder (1) which can have very favorable effects on the health of the pregnant woman and her unborn child which cannot be found with any conventional maternity binder.

Fig. 1

EP 0 348 355 A1

# Description

## Maternity Binder

FIELD OF THE INVENTION

This invention relates to a maternity binder

BACKGROUND OF THE INVENTION

Hitherto, it has been known to use a maternity binder to provide thermal insulation and protection of the abdominal region of a pregnant woman. There are various kinds of such binder, including binders of the so-called belly-band type and those of the lengthy cloth-made sash type.

(Problem of the Prior Art)

However, any one of these conventional maternity binders can merely provide thermal insulation and protection of the abdomen of the pregnant woman when it is wrapped around the abdomen, and no further effect can be expected of it. None of the conventional maternity binders function as a positive means for improvement of the health of the pregnant woman.

Therefore, it is a primary object of the invention to provide a maternity binder which has an additional and excellent function such that it gives favorable effects on the health of the pregnant woman and her unborn child which cannot be found with any conventional maternity binder.

SUMMARY OF THE INVENTION

In view of the fact that recently in the medical and other fields it has been clarified that far-infrared rays exhibit good performance characteristics when applied to human body, the present invention is directed to solving the aforementioned problem with the prior art by arranging that far-infrared rays are radiated toward the abdomen of the pregnant woman.

According to a first aspect of the invention, a maternity binder comprising a band 2 provided with a far-infrared ray emitting element 5 is provided.

According to a second aspect of the invention, in the maternity binder according to the above mentioned first aspect, the far-infrared ray emitting element 5 comprises an aluminum sheet 7 for reflecting far-infrared rays and a far-infrared ray emitting ceramics layer 8 formed on one side of the aluminum sheet 7.

According to a third aspect of the invention, in the maternity binder according to one of the above mentioned first and second aspects, the band 2 is provided with a pocket 6 for removably housing the far-infrared ray emitting element 5 therein.

According to the maternity binder of the above noted arrangement, far-infrared rays can constantly be radiated toward the abdomen of the pregnant woman around which the band 2 are wrapped.

The far-infrared rays transmit through the surface of the abdomen of the pregnant woman into the subcutaneous depth thereof so that favorable thermal effects, increased metabolism, and/or enhanced blood circulation can be obtained with respect to the uterus and other organs and cellular tissues, and even to the unborn baby and forewaters. Thus, the maternity binder of the invention is helpful for the enhancement of health of the pregnant woman and for the growth of her unborn child and, after childbirth, it can further physical recovery of the user.

In the arrangement in which the aluminum sheet 7 is provided on one side of the far-infrared ray emitting element 5, the ceramics layer 8 which emits infrared rays may be positioned on the inner side of the aluminum sheet 7, whereby the far-infrared rays emitted in a direction opposite to the pregnant woman are reflected from the aluminum sheet 7 so that they are effectively applied to the abdomen of the user, the efficiency of their radiation being thus enhanced. Furthermore, the aluminum sheet 7 concurrently serves to interrupt various electromagnetic waves emitted from external sources toward the abdomen of the user, and thus electromagnetic waves emitted from a television set, a microwave oven, or the like which are harmful to the pregnant woman and her unborn child can be conveniently prevented from being radiated to the abdomen.

In the arrangement in which the band 2 is provided with a pocket 6 for housing the far-infrared ray emitting element 5 therein, the far-infrared ray emitting element 5 can be readily attached to and removed from the band 2, so that one far-infrared ray emitting element 5 may be used with one or another of a plurality of bands 2. When washing of the band 2 is required, the far-infrared ray emitting element 5 is removed from the pocket 6 so that the band 2 alone can be washed. Therefore, no possible quality deterioration of the far-infrared ray emitting element 5 can result from washing.

According to the invention, as described above, the band of the maternity binder is equipped with a far-infrared emitting element; therfore, far-infrared rays can be efficiently radiated directly toward the abdomen of the pregnant woman to provide their favorable effects, such as thermal insulation, improved blood circulation, and increased metabolism, which can be extended not only to the pregnant woman, but also to her unborn child. Furthermore, such favorable effects can be obtained in the form of accelerated uterine recovery after childbirth. None of these favorable effects could be obtained with any conventional maternity binder.

With the arrangement of the invention in which the far-infrared emitting ceramics layer is positioned on one side of the aluminum sheet, far-infrared rays emitted from the ceramics layer can be reflected by the aluminum sheet toward the abdomen of the pregnant woman, it being thus possible to obtain improved radiation efficiency and further to ensure good contribution toward sound growth of the unborn child since the aluminum sheet can interrupt harmful electromagnetic waves emitted from such sources as TV and microwave oven.

According to the arrangement in which the band is equipped with a pocket for removably housing the far-infrared ray emitting element therein, the emitting element can be readily removed from the band when washing is required of the band, it being thus prevented from possible damage due to washing. This enables prolonged use and assures greater serviceability value.

## BRIEF DESCRIPTION OF THE DRAWINGS

Fig. 1 is a perspective view showing one embodiment of the invention;

Fig. 2 is an enlarged section taken along line X-X in Fig. 1; and

Figs. 3 (a) and 3 (b), and Fig. 4 are perspective views showing other embodiments of the invention.

## DETAILED DESCRIPTION OF THE INVENTION

One embodiment of the invention will now be described.

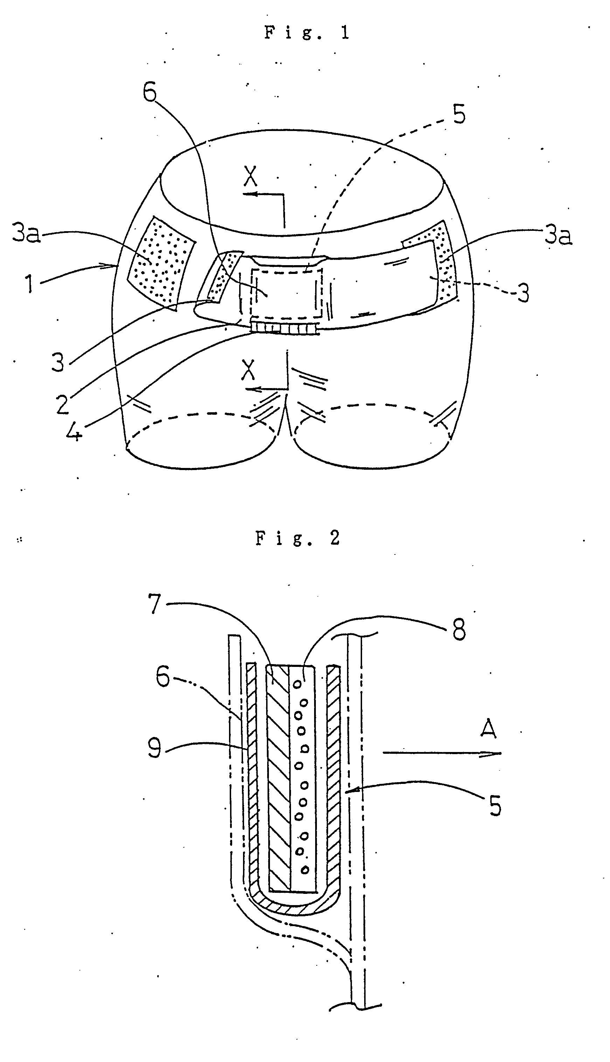

In Fig. 1, numeral 1 designates a girdle to be worn by a pregnant woman for warmth keeping purposes and otherwise with respect to the abdomen and waist. Numeral 2 designates a cloth-made band in the form of a belt having a length of 30 to 40 cm which serves as a supplementary belt for the girdle 1 and a median portion of which is sewn at its lower edge 4 to the girdle 1. Shown by 3, 3 are magic tape pieces secured to the back of the band 2 at opposite ends thereof which are detachably engageable with broader magic tape pieces 3a, 3a secured to the front side of the girdle 1 at opposite ends thereof. These magic tape pieces are convenient for adjustment of the tightness or looseness of the band 2 according to the body size of the pregnant woman.

Numeral 6 designates a top open pocket formed generally centrally in the band 2. Numeral 5 designates a far-infrared ray emitting element removably housed in the pocket 6 which, as Fig. 2 shows, comprises an aluminum sheet 7 having a ceramics layer 8 formed on its surface which is contained in a protetion cover 9. It is noted that the ceramics layer 8 comprises a coating of a powdery ceramic material composed principally of zirconium, alumina, silica and the like which radiates far infrared rays as electromagnetic waves of a wave length of not more than 15 μm at ordinary temperatures.

The construction of the present embodiment has now been described. Nextly, the function of the embodiment will be explained.

When the pregnant woman wears the girdle 1 which, as Fig. 1 shows, has a far infrared ray emitting element 5 housed in the pocket 6 of the band 2, the girdle 1 provides good thermal insulation and protection of her abdomen and, moreover, the far infrared ray emitting element 5, being positioned in front of the abdomen of the pregnant woman, radiates far infrared rays directly onto her abdomen. Particularly because the aluminum sheet 7 which reflects the far infrared rays in the direction of A is present on the outer side of the ceramics layer 8 which radiates the far infrared rays, the far infrared rays are radiated very effectively.

Far infrared rays, as they are radiated from the far infrared ray emitting element 5 towrad the abdomen of the pregnant woman, provide good thermal effect for the abdomen since they transmit through the surface of the abdomen into the depth thereof, and further the far infrared rays serve to activate the cellular tissue and the like and promotes blood circulation. All these have favorable effects on the uterus and other organs and cellular tissues, thus helping in the promotion of health of the pregnant woman. The far infrared ray radiation serves to correct any abnormality in hormonal secretion during pregnancy thereby to stabilize the mental condition of the pregnant woman. In addition, the far infrared ray radiation has a favorable effect on the growth of the unborn child as it acts on the unborn child and forewaters.

The aluminum sheet 7 of the far infrared emitting element 5 has a function to reflect various electromagnetic waves radiated from an external source toward the abdomen of the pregnant woman to thereby interrupt them. This eliminates the possibility of harmful electromagnetic waves from such sources as television and microwave oven being radiated toward the abdomen of the pregnant woman, which fact makes the maternity band of the invention beneficial all the more for the health of the pregnant woman and her unborn child.

The girdle 1 equipped with the band 2 may be worn not only during pregnancy, but also during a postpartum period in which considerable fatigue is involved. When it is worn after childbirth, far-infrared ray radiation promotes the uterine recovery of the user and activates mother-milk delivery and urinary excretion.

When the girdle 1 and band 2 are to be washed, the far-infrared emitting element 5 should be removed from the pocket 6. By doing so it is possible to conveniently avoid possible removal or loss of the ceramics layer 8 of the far-infrared emitting element 5. Thus, the far-infrared emitting element 5 can be used for a long period of time. It is also possible to use one far-infrared emitting element 5 by housing it in the pocket of another band, which fact adds to the convenience of use of the far-infrared emitting element 5.

In the above described embodiment, the maternity binder of the invention is formed as a supplementary band mounted to the girdle, but it is not intended that the invention is limited to such form.

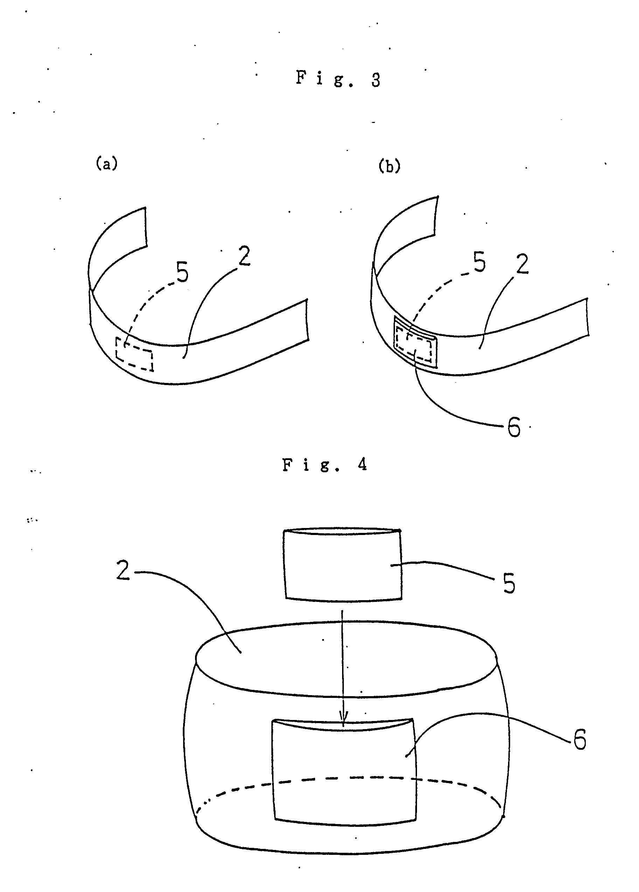

For example, as Fig. 3 (b) shows, a band 2 separate from a girdle or the like has a far-infrared emitting element 5 contained therein. The band 2 may be used in conjunction with the girdle by being mounted thereto, or may be used alone by being wrapped around the abdominal region. The band 2 is not particularly limited in length. For example, the band 2 may be in the form of a lengthy cloth band having a length of several meter or so. The band 2 of the invention embraces, for example, one formed in such a ring shape as shown in Fig. 3 (b). In this way, the band 2 of the invention is not limited in shape or size, nor is it specifically limited in material.

Likewise, the far-infrared emitting element 5 is in no way limited to such construction as noted in the above described embodiment. In the maternity binder as claimed in claim 1, the far infrared emitting element 5 is not particularly limited in size or configuration, provided that it can radiate far infrared rays.

The band 2 of the invention should not necessarily have a pocket 6. For example, as Fig. 3 (a) shows, the far-infrared emitting element 5 may be incorporated into the band 2 by sewing.

Other details of the invention may be changed, varied, and modified in design as desired within the envisaged scope of the invention.

**Claims**

1. A maternity binder comprising a band 2 provided with a far-infrared ray emitting element 5.

2. The maternity binder according to claim 1 wherein the far-infrared ray emitting element 5 comprises an aluminum sheet 7 for reflecting far-infrared rays and a far-infrared ray emitting ceramics layer 8 formed on one side of the aluminum sheet 7.

3. The maternity binder according to claim 1 or 2 wherein the band 2 is provided with a pocket 6 for removably housing the far-infrared ray emitting element 5 therein.

4. The maternity binder according to one of claims 1 to 3 wherein the band 2 is attached as a supplementary belt to the front portion of a girdle 1.

Fig. 1

Fig. 2

Fig. 3

(a)

(b)

Fig. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-4 557 268 (I.H. MADDUX) <br> * Column 1, lines 53 - 68; figures 1-4 * | 1,4 | A 41 C 1/10 <br> A 61 N 5/06 |
| A | FR-A-2 428 435 (B. COURET) <br> * Whole document * | 1-3 | |
| A | EP-A-0 228 537 (ELEC SYSTEM) <br> * Claims 1-6; figures 1,2 * | 1 | |
| A | DE-C- 874 059 (M. FUCHS) <br> * Claims 1-3; figures 1-3 * | 1 | |

TECHNICAL FIELDS
SEARCHED (Int. Cl.4)

A 41 C
A 61 F
A 61 N
A 61 B

The present search report has been drawn up for all claims

| Place of search | Date of completion of the search | Examiner |
|---|---|---|
| THE HAGUE | 12-09-1989 | GARNIER F.M.A.C. |